Europäisches Patentamt

European Patent Office

Office européen des brevets

(11) Numéro de publication: **0 269 511 B1**

# (12) FASCICULE DE BREVET EUROPEEN

(45) Date de publication de fascicule du brevet: **13.01.93** (51) Int. Cl.5: **G01N 31/12**, G01N 33/24

(21) Numéro de dépôt: **87402593.5**

(22) Date de dépôt: **18.11.87**

(54) **Procédé et dispositif de détermination des teneurs d'au moins deux éléments choisis parmi le carbone, l'hydrogène, le soufre et l'azote d'au moins deux fractions d'un échantillon de matière organique.**

(30) Priorité: **25.11.86 FR 8616409**

(43) Date de publication de la demande:
**01.06.88 Bulletin 88/22**

(45) Mention de la délivrance du brevet:
**13.01.93 Bulletin 93/02**

(84) Etats contractants désignés:
**BE DE ES GB IT NL**

(56) Documents cités:
FR-A- 2 376 414
GB-A- 2 116 320
US-A- 3 861 874

(73) Titulaire: **INSTITUT FRANCAIS DU PETROLE
4, Avenue de Bois Préau
F-92502 Rueil-Malmaison(FR)**

(72) Inventeur: **Bigois, Maurice
23, quai Victor Augagneur
F-69003 Lyon(FR)**
Inventeur: **Novat, Christian
Le Vieux Jonc Impasse de la Source
F-01240 Saint Paul de Varay(FR)**
Inventeur: **Le Perchec, Pierre
99, rue Pierre Corneille
F-69003 Lyon(FR)**
Inventeur: **Fixari, Bernard
3, rue Le Corbusier
F-38200 Vienne(FR)**

Rank Xerox (UK) Business Services

## Description

La présente invention concerne un procédé permettant de déterminer la quantité d'au moins deux éléments choisis parmi le carbone, l'hydrogène, le soufre et l'azote d'au moins deux fractions d'un échantillon de matière organique et l'appareil pour la mise en oeuvre du procédé.

Elle s'applique à la caractérisation d'échantillons de nature variée tels que les matières organiques fossiles (pétroles, charbons, bitumes, sables bitumineux, schistes bitumineux, tourbes, matières végétales), les matières organominérales (par exemple, phosphates, humus,...), les polymères organiques naturels (par exemple, caoutchouc, gomme xanthane,...), les polymères de synthèse (par exemple, polyéthylène, polystyrène, polychlorure de vinyle, polyuréthanes). Elle est particulièrement utilisée dans l'industrie pétrolière et du charbon, notamment lors de la caractérisation des effluents de raffinage en relation avec la sévérité des procédés mis en jeu (hydrotraitements, viscoréduction, etc.).

La caractérisation des matières hydrocarbonées naturelles ou synthétiques contenant éventuellement d'autres éléments (hétéroéléments, matières inorganiques, métaux), se réalise par l'application de techniques thermoanalytiques homologuées par l'American Society for Testing Materials (normes ASTM). Les normes D86, D1160, D2887 font références aux techniques de TBP (True Boiling Point) pour la détermination des distributions des produits volatils en opérant de façon préparative (100 ml) sur des échantillons de matières naturelles.

La caractérisation des fractions liquides est réalisable par une distillation simulée qui utilise un chromatographe en phase vapeur, a température programmée, couplé à une banque de donnée standardisée des points d'ébullition de paraffines. Cette technique analytique fournit une analyse précise de la distribution des fractions distillables jusqu'à 580-600°C (point d'ébullition de paraffines en C55-C60) pour une température de colonne de l'ordre de 350°C, mais ne fournit aucune indication sur la fraction lourde. Une amélioration récente permet d'atteindre des points d'ébullition de paraffines en C100-C120 : 700 à 800°C, cela pour des températures de colonne de 430°C.

La méthode thermogravimétrique consiste à mesurer la perte de poids d'un échantillon au cours d'un traitement thermique progressif ou pendant un temps de chauffage isotherme prédéterminé. Cette opération nécessite une thermobalance ne pouvant être mise en oeuvre que sous de faibles débits gazeux. L'analyse reste incomplète car seule la perte de poids peut être déterminée (matières vaporisables) précisément en continu, mais aucune information sur la matière résiduelle ne peut être connue, ainsi que lors de la distillation simulée. De même, le profil de perte de poids au-delà de 400°C de température du four n'implique pas une analyse précise de la distribution des éléments constitutifs en fonction de l'aptitude au craquage et à la cokéfaction de la matière hydrocarbonée.

En présence de matières naturelles (à poids moléculaires élevés) contenant des hétéroéléments et des métaux, ces techniques perdent de leur fiabilité particulièrement pour les produits distillables au-delà de 530°C, d'où il ressort la nécessité de disposer de méthodes plus fiables et quantitatives.

Le brevet US 3880 587 concerne un procédé et un appareil dans lequel on chauffe un échantillon liquide à au moins 300°C au cours d'une première étape en l'injectant dans la colonne d'un chromatographe en phase gazeuse, et les effluents sont oxydés dans un premier four ; on détermine ainsi par une double détection la quantité de $CO_2$ et de $SO_2$ produits à partir des matières volatiles. On analyse ensuite la fraction dite résiduelle en balayant la colonne chromatographique à contre courant avec de l'oxygène dans le but de chasser de cette colonne le résidu moins volatil, que l'on chauffe dans un autre four à combustion. Dans ces conditions, l'analyse du résidu ne peut être qu'incomplète dans la mesure où l'adsorption de produits lourds non volatils sur la colonne chromatographique, lors de la première étape, quel que soit le type de phase adsorbante qu'elle contient, est pratiquement irréversible. Par ailleurs, il n'est décrit ni suggéré l'analyse simultanée de l'eau résultant de la combustion des composés hydrocarbonés.

Dans le brevet FR 2376 414, on procède à une pyrolyse d'un sédiment géologique sous gaz inerte, on réalise une combustion de l'effluent de manière continue au cours de la pyrolyse et l'on détecte et mesure d'une part les hydrocarbures, et d'autre part, les composés soufrés sous forme $SO_2$. L'analyse est cependant incomplète puisqu'on ne réalise pas la combustion du résidu. Par ailleurs, bien que l'on détecte le soufre des hydrocarbures par combustion de l'effluent, il n'est pas réalisé une détection et une analyse des produits d'oxydation des hydrocarbures et notamment de l'eau.

Le brevet US 3 861 874 décrit l'analyse des composés volatils et non volatils d'un échantillon de matière organique de façon à obtenir une information quantitative de tout l'effluent en fonction de la température d'ébullition de ses constituants. On envoie un courant de gaz inerte sur un échantillon et on procède à un chauffage à une vitesse prédéterminée jusqu'à ce que la majorité des composés volatils soit déplacée. Ces composés sont ensuite convertis en $CO_2$ grâce à un catalyseur d'oxyde cuivrique dans un tube à combustion et la teneur en $CO_2$ est mesurée par un détecteur à conductivité thermique.

Ce système d'oxydation présente l'inconvénient d'être peu fiable au cours du temps : en effet, on observe une décroissance de l'activité catalytique au cours de l'analyse et il est précisé qu'il est nécessaire de réoxyder l'oxyde de cuivre dans le tube à combustion et de réduire le cuivre métallique dans le piège à oxygène disposé avant le détecteur à conductivité thermique.

Par ailleurs, l'art antérieur peut être illustré par les brevets GB-A-2 116 320, GB-A-2 173 305, GB-2 066 462 et FR-2 339 173.

Dans l'ensemble de ces documents, il n'est décrit, ni enseigné de détecter au cours du temps d'analyse et de manière sensiblement simultanée, avec des détecteurs spécifiques, au moins deux éléments choisis parmi le carbone, l'hydrogène, le soufre et l'azote dans au moins deux fractions d'un échantillon de matière organique, l'une volatile, l'autre non volatile.

A ce jour, à notre connaissance et malgré l'importante littérature émanant notamment des raffineurs, très demandeurs d'analyses susceptibles de répondre rapidement et avec précision à des études de cinétique et de valorisation dans les procédés de raffinage (hydrotraitement par exemple), il n'est pas connu d'art antérieur qui réponde notamment aux objectifs suivants :

- disposer d'un bilan global quantitatif en carbone, hydrogène et/ou soufre, voire azote, sur au moins deux fractions, et en particulier sur une fraction volatile et sur une fraction résiduelle,
- obtenir une information fiable et rapide de la distribution des paramètres étudiés, tout en bénéficiant d'une précision acceptable et comparable aux méthodes prises séparément, et
- bénéficier de la simultanéité des analyses en ligne en carbone, hydrogène, soufre et/ou azote, afin de calculer les valeurs de leurs rapports respectifs, par exemple $H_{/C}$, $S_{/C}$, $H_{/S}$, en fonction de la température de chauffage ou de tout autre paramètre, par exemple pour la détermination des seuils de température de craquage, de déshydrogénation, de désulfuration et de dénitrogénation.

Le procédé selon l'invention remédie aux inconvénients de l'art antérieur et répond aux objectifs et aux problèmes soulevés. Il permet plus particulièrement de déterminer les teneurs d'au moins deux éléments choisis parmi l'hydrogène, le carbone, le soufre et l'azote, contenus dans au moins deux fractions d'un échantillon de matière organique ou de matière inorganique.

Ce procédé comprend une étape de chauffage sous flux d'atmosphère inerte à une température $T_1$ au plus égale à 700° C dans une première zone de chauffage, de façon à réaliser une étape de vaporisation-distillation délivrant un effluent gazeux de la fraction vaporisée et un résidu, une étape de combustion dudit effluent gazeux en présence d'un catalyseur d'oxydation dans une zone de combustion et une étape de combustion du résidu par un gaz contenant de l'oxygène moléculaire délivrant en présence d'un catalyseur d'oxydation un produit d'oxydation dudit résidu ; ce procédé est caractérisé en ce que :

a) on effectue ladite étape de combustion dudit effluent gazeux en présence d'un gaz pauvre en oxygène dans des conditions telles que l'on forme des premiers composés d'oxydation desdits éléments contenus dans ledit effluent et comprenant essentiellement de l'anhydride carbonique, de la vapeur d'eau, de l'anhydride sulfureux et/ou de l'oxyde d'azote et, on détecte de manière sensiblement simultanée au moyen de détecteurs spécifiques deux au moins desdits premiers signaux représentatifs desdits premiers composés d'oxydation ;

b) on effectue ladite étape de combustion dudit résidu par un gaz pauvre en oxygène moléculaire dans des conditions telles que l'on forme des seconde composés d'oxydation desdits éléments contenus dans ledit produit d'oxydation et comprenant essentiellement de l'anhydride carbonique, de la vapeur d'eau, de l'anhydride sulfureux et/ou de l'oxyde d'azote, et on détecte de manière sensiblement simultanée au moyen desdits détecteurs deux au moins des seconds composés d'oxydation contenus dans ledit produit d'oxydation du résidu de façon à obtenir des seconds signaux représentatifs desdits seconds composés, et

c) on déduit desdits premiers et seconds signaux lesdites teneurs d'au moins deux éléments respectivement de la fraction vaporisée et du résidu.

On obtient ainsi une information fiable, rapide, précise, reproductible et quantitative.

Le terme d'étape de vaporisation-distillation a été utilisé de manière générale. Il regroupe en effet l'ensemble des composés volatils à une température donnée. Ceux-ci peuvent provenir aussi, au moins en partie, d'une phase de pyrolyse, si la température de chauffage est élevée, qui entraîne le craquage des molécules de haut point d'ébullition en entités plus petites et de point d'ébullition plus faible. On peut donc mettre en évidence, suivant un mode particulier de réalisation du procédé, au moins trois fractions dans l'analyse d'un échantillon : une fraction résultant de la vaporisation-distillation, une fraction résultant du craquage par pyrolyse et une fraction résiduelle.

On peut même mettre en évidence 4, 5,...n fractions suivant le type de produit étudié ou suivant l'information analytique recherchée.

On peut bien évidemment ne s'intéresser qu'à un élément ou à un couple d'éléments suivant la nécessité, toutes les combinaisons entre les divers éléments étant possibles. On peut aussi n'analyser que trois éléments, C, H et S ou N si l'échantillon ne présente que peu ou pas de soufre ou d'azote. De préférence, on détermine cependant l'hydrogène et au moins un élément choisi parmi le carbone, le soufre et l'azote.

Suivant un mode de réalisation, on chauffe ledit échantillon sous atmosphère inerte à une température $T_1$, de préférence de la température ambiante jusqu'à la température $T_1$ à une vitesse d'augmentation de température comprise entre 1 et 100°C/mn et de préférence entre 10 et 50°C/mn, et l'on détecte de manière sensiblement simultanée lesdits premiers composés d'oxydation de façon à obtenir lesdits premiers signaux en fonction de la température.

Suivant un autre mode de réalisation du procédé, la température $T_1$ peut être maintenue constante durant l'étape de vaporisation-distillation. Elle peut être aussi atteinte par chauffage par paliers successifs, ou par palier(s) puis par programmation de température ou inversement, toutes les combinaisons étant possibles et étant fonction du type de produit à analyser. On peut dans les conditions ci-dessus obtenir lesdits premiers signaux en fonction de la température de la première zone de chauffage.

La température de chauffage $T_1$ est généralement au plus égale à 700°C et de manière avantageuse comprise entre 100°C et 600°C.

On effectue généralement la combustion du résidu dans une zone de chauffage qui est avantageusement la même que celle où s'est effectué le chauffage en atmosphère inerte, à une température $T_2$ au plus égale à 1100°C, et avantageusement au moins égale à $T_1$. Cette température $T_2$ est fonction du type de résidu à oxyder et elle peut être soit maintenue constante durant tout le cycle de combustion, soit atteinte par paliers successifs, soit atteinte à une vitesse d'augmentation de la température généralement comprise entre 1 et 100°C et de préférence 10-50°C/mn.

L'oxydation, aussi bien de l'effluent gazeux de vaporisation-distillation, que du résidu, s'effectue avantageusement en atmosphère pauvre en oxygène (1 à 15 % en volume en général), de préférence avec un mélange de gaz inerte sec (azote excepté) et d'oxygène sensiblement pur, débarrassé des traces de carbone, hydrogène, soufre et azote, présentes sous forme d'impuretés organiques ou minérales.

Oxydant ainsi avec un gaz pauvre en oxygène, on favorise la transformation spécifique du soufre en anhydride sulfureux $SO_2$ plutôt qu'en anhydride sulfurique $SO_3$. On a obtenu d'excellents résultats en présence d'hélium contenant de 1 à 15% en volume d'oxygène et de préférence de 2 à 5%.

Le débit des gaz utilisés est généralement important pour éviter les phénomènes d'inertie, ce qui facilite la vaporisation de l'échantillon et permet d'obtenir des mesures reproductibles des divers éléments, avec une très bonne précision puisque les interférences entre gaz détectés sont minimisées, rapides (en général moins de 60 mn) et en temps réel.

L'oxydation de l'effluent gazeux de vaporisation-distillation s'effectue dans une zone d'oxydation chauffée en communication avec la première zone de chauffage sous atmosphère inerte. Cette zone d'oxydation comprend un four qui renferme au moins un catalyseur d'oxydation, CuO de préférence. Cette zone peut être soumise à un gradient de température positif et peut être divisée par exemple en trois parties, de l'amont vers l'aval, en fonction de l'écoulement des gaz :

- une première partie contenant par exemple, au moins en partie, de la laine de silice chauffée à une température $T_{01}$ au moins sensiblement égale à la température $T_1$, et de préférence comprise entre 600° et 800°C,
- une deuxième partie contenant le catalyseur, CuO par exemple, à une température $T_{02} > T_{01}$ comprise entre 700 et 900°C, de préférence entre 750 et 850°C correspondant à la température optimum d'activité pour le catalyseur, et
- une troisième partie chauffée à une température $T_{03} > T_{02}$ comprise entre 800 et 1000°C, de préférence entre 850 et 950°C, assurant la simultanéité de la vitesse de diffusion des gaz.

Dans ces conditions de passage sur le catalyseur et à ces niveaux de température $T_{01}$, $T_{02}$ et $T_{03}$, on assure le maintien de l'équilibre des gaz de combustion en faveur de l'anhydride carbonique et de l'anhydride sulfureux, on minimise la formation de monoxyde de carbone et d'anhydride sulfurique tout en ne dégradant pas le catalyseur, et on assure une vitesse de diffusion sensiblement égale de tous les gaz.

Le débit $d_2$ de gaz inerte dans la première zone de chauffage de l'échantillon est généralement compris entre 100 et 1000 ml/mn et de préférence compris entre 200 et 400 ml/mn.

Le débit $d_1$ de mélange de gaz pauvre en oxygène ou comburant pour l'oxydation de l'effluent gazeux de vaporisation est généralement compris entre 50 et 400 ml/mn, et de préférence entre 100 et 250 ml. On s'arrangera suivant un mode particulier de réalisation de l'invention, lors de l'étape a) du procédé, phase de vaporisation-distillation et phase d'oxydation en continu de l'effluent gazeux de la fraction vaporisée, pour que $d_2 > d_1$ et qu'ainsi, il n'y ait sensiblement pas d'oxygène dans la première zone de chauffage en

EP 0 269 511 B1

atmosphère inerte.

Lors de l'étape d'oxydation du résidu, on peut stopper l'arrivée de l'hélium seul et on peut le remplacer par le mélange de gaz comburant de sorte que les débits en gaz comburant introduits sont sensiblement égaux à la somme des débits $d_1$ et $d_2$ introduits lors de l'étape a) du procédé, ce qui présente l'avantage de ne pas perturber la mesure.

Les composés d'oxydation de l'effluent gazeux résultant du chauffage sous atmosphère inerte jusqu'à la température $T_1$ sont détectés simultanément et analysés en ligne pendant l'étape de chauffage par un détecteur d'eau, un détecteur à $CO_2$, un détecteur à $SO_2$ et un détecteur à oxydes d'azote (NO), montés en série ou en parallèle de préférence en série, ce qui présente l'avantage d'une analyse plus quantitative de tout l'effluent. Le détecteur d'eau est avantageusement placé à la sortie de la zone d'oxydation car l'eau diffuse moins que les autres composés d'oxydation et circule donc moins rapidement.

La zone de détection est généralement thermostatée par exemple vers 70°C environ et sous débit gazeux pour :

- éviter par variation de température des cellules de mesure, une dérive de la sensibilité du système de mesure au cours d'une analyse ou d'analyses successives,
- volatiliser l'eau formée au cours de la combustion afin, d'une part, de mesurer simultanément les signaux (issus des photomètres respectifs) représentatifs des concentrations de l'eau et le dioxyde de carbone mis en mémoire par le système d'acquisition de données, et, d'autre part, d'éviter les condensations d'eau dans les circuits gazeux pouvant provoquer des effets de "mémoire".

Les composés d'oxydation du résidu sont détectés simultanément et analysés en ligne par les mêmes détecteurs que ceux ci-dessus.

Les détecteurs utilisés dans les deux cas sont ceux décrits dans la littérature et de préférence, les détecteurs à infra-rouge pour l'eau et l'anhydride carbonique, le détecteur à ultraviolet pour l'anhydride sulfureux, et le détecteur à chimiluminescence (NO) ou le catharomètre ($N_2$) pour l'azote, ce dernier pouvant être avantageusement précédé d'un piège contenant du cuivre réduit. Chaque détecteur est spécifique d'un composé étudié et présente l'avantage d'une détection sensiblement linéaire et simultanée. La catharométrie suppose cependant une élimination du $CO_2$, $H_2O$ et $SO_2$ préalable au passage dans le détecteur.

A partir d'un échantillon de très petite taille généralement inférieure à 50 mg et de préférence comprise entre 1 et 30 mg, on détermine simultanément une courbe (pyrogramme) représentative de chaque élément choisi (C, S, H et/ou N), dont on désire connaître la distribution en fonction de la température de chauffage sur au moins deux fractions, la première étant plus volatile que la seconde des deux fractions de l'échantillon.

Sur au moins une des deux fractions ci-dessus, et de préférence sur la fraction la plus volatile, on pourra selon un autre mode de l'invention, définir au moins deux signaux représentatifs d'un ou de plusieurs des éléments, de préférence d'au moins deux des éléments, choisis dans le groupe formé par le carbone, l'hydrogène, le soufre et l'azote, ces signaux correspondant à au moins deux intervalles de temps définis successifs.

Selon un autre mode de l'invention, on peut transformer l'échelle de température de chauffage en atmosphère inerte et/ou en atmosphère oxydante, en une échelle de température d'ébullition, déterminée à partir du chauffage, dans les mêmes conditions que l'étape a) du procédé selon l'invention, de composés de référence purs, convenablement choisis. Les n-alcanes sont généralement utilisés dans l'industrie pétrolière, les aromatiques possédant un nombre de cycles condensés de 1 à 7 ou plus, ou les composés polaires (acides, alcools, amines...) pouvant être aussi utilisés. Dans ces conditions, on peut par exemple obtenir l'équivalent d'une distillation simulée à partir de la distribution en carbone de la fraction volatile, en fonction de la température d'ébullition de ses constituants.

A partir des diverses répartitions en C, S, H et M, on peut à tout moment calculer les valeurs des rapports respectifs ou leurs inverses $C_{/H}$, $C_{/S}$, $H_{/S}$, $C_{/N}$, $H_{/N}$, $S_{/N}$, et les corréler entre eux ou en fonction d'autres paramètres, comme on le verra ci-après, dans les applications.

L'invention concerne aussi l'appareil utilisable pour la mise en oeuvre du procédé : il comprend en combinaison :

- une enceinte **(2)**,
- des moyens de chauffage **(1)** de ladite enceinte,
- des moyens de supports **(3)** dudit échantillon dans ladite enceinte,
- des moyens d'alimentation **(21a, 22a, 24, 5)** en gaz inerte reliés à ladite enceinte, qui sont adaptés à produire en combinaison avec les moyens de chauffage ledit effluent gazeux de la fraction vaporisée,
- des moyens d'alimentation **(21b, 22b, 24b, 14)**, en mélange de gaz comburant reliés à des moyens de combustion définis ci-dessous,

- des moyens de combustion **(30, 2b)** reliés à ladite enceinte **(2)** et adaptés d'une part à délivrer lesdits premiers composés d'oxydation desdits éléments à partir de l'effluent gazeux et, d'autre part, à délivrer lesdits seconds composés d'oxydation à partir dudit produit d'oxydation du résidu,
- des moyens d'alimentation **(21b, 22a, 24a, 5)** en mélange de gaz comburant reliés à ladite enceinte qui sont adaptés à produire ledit produit d'oxydation du résidu,
- des moyens de détection spécifiques **(16, 17, 18, 19)** adaptés à détecter simultanément deux au moins desdits premiers composés d'oxydation, puis deux au moins desdits seconds composés d'oxydation, et reliés auxdits moyens de combustion **(2b, 30)**,
- des moyens de traitement **(20)** reliés auxdits moyens de détection et adaptés à traiter lesdits premiers et seconds signaux.

L'invention sera mieux comprise au vu de la figure **1** schématique suivante qui illustre à titre non limitatif un mode de réalisation particulier de l'invention, au vu de la figure 2 qui montre un exemple de profil de température et au vu des figures 3, 4 et 5 qui montrent par exemple les signaux correspondant respectivement au $CO_2$, $H_2O$ et $SO_2$.

Le dispositif comprend des moyens de chauffage **1** avec un four **1a** chauffant par tout moyen connu, par exemple un enroulement d'une résistance **1a**, la première partie **2a** d'une enceinte **2** de forme avantageusement tubulaire et allongée, ou tube à combustion.

Le prélèvement analytique de l'échantillon de masse par exemple inférieure à 10 mg est placé dans une nacelle **3** en platine ou porcelaine et est introduit dans une allonge **4** en quartz balayée par un courant d'hélium ou suivant la deuxième étape du procédé, par un courant d'hélium et d'oxygène apporté par une ligne **5**.

L'allonge est installée dans l'enceinte **2a** avec la nacelle **3** placée entre deux remplissages de laine de quartz. Cette allonge **4** comprend par exemple un tube en quartz qui s'évase, afin d'éviter tout retour de gaz oxygéné vers l'enceinte contenant la nacelle porteuse de l'échantillon, en aval, sous forme d'une corolle, et par lequel est introduit un couple thermoélectrique de contrôle **7** qui contrôle au niveau de la nacelle **3** la température de vaporisation-distillation et pyrolyse de l'échantillon à analyser.

L'allonge **4** est fixée à une extrémité de l'enceinte **2a** à l'aide d'une pièce en laiton **8a** qui permet en outre l'arrivée du mélange de gaz comburant hélium et oxygène par une ligne **14**. Cette disposition permet un meilleur balayage de l'extérieur de l'allonge **4** et par conséquent, évite la rétrodiffusion des composés. Elle permet aussi de réchauffer le mélange de gaz comburant au contact de l'allonge.

Le chauffage du four **1a** est assuré par un automate à microprocesseur **10** qui commande les moyens de contrôle **7**, de régulation et de programmation de température **9**. Cet automate gère en outre les différents paramètres contrôlant le procédé (définition de la température, vitesse de montée en température, définition des durées des isothermes de température, action sur les commandes externes (notamment les vannes électromagnétiques).

Une sonde (non montrée sur la figure) disposée de préférence dans le débit gazeux d'élution est reliée aux moyens de contrôle **7**.

Le tube de combustion **2** qui est chauffé dans sa première partie par le four **1a** entre 0° et 1100°C, présente une deuxième partie **2b** (deuxième enceinte) en communication avec la première **2a.** Cette deuxième partie **2b** du tube **2** distincte de la première est chauffée par un deuxième four **(30)** dont la température peut atteindre 1000°C et peut être contrôlée, régulée et programmée par des moyens connus non représentés et gérés par l'automate à microprocesseur **10**.

Elle comprend le catalyseur **11** d'oxydation, par exemple de l'oxyde cuivrique CuO, qui est généralement chauffé entre 700 et 900°C et que l'on place entre deux quantités de laine de silice **25.**

Cette deuxième enceinte chauffée **2b** peut être avantageusement divisée en trois zones ; la première **27** remplie de laine de silice est chauffée par exemple à $T_{01}$ égale à 750°C, la seconde **28** contient le catalyseur et est chauffée par exemple à $T_{02}$ égale à 850°C et la troisième **29** est chauffée par exemple à $T_{03}$ égale à 950°C et peut contenir une baguette pleine et inerte **31**, de préférence en quartz et d'un diamètre tel que les composés d'oxydation sont portés à la plus haute température possible au contact des parois chaudes, ce qui permet la transformation de l'anhydride sulfurique formé éventuellement au contact du catalyseur en anhydride sulfureux et le transfert du mélange des gaz effluents d'oxydation dans leur composition initiale, au niveau des détecteurs.

Une ligne **12** d'évacuation, par exemple en téflon, relie l'autre extrémité de la deuxième partie **2b** du tube à combustion **2** aux divers détecteurs.

L'étanchéité aux extrémités de cet ensemble vis-à-vis de l'atmosphère est assurée par des joints toriques **13a** et **13b** supportés par des pièces en laiton **8a** et **8b**.

Une ligne **15** d'hélium supplémentaire disposée à la sortie du tube à combustion **2b** alimente la zone de détection à un débit supplémentaire pouvant atteindre jusqu'à 400 ml/mn et de préférence 80 à 150 ml/mn pour qu'il n'y ait pas de dépression dans le dispositif, pour que les effluents d'oxydation ne soient pas retenus et pour que les signaux soient délivrés en temps réel.

Les gaz sont distribués, par exemple à partir de bouteilles de gaz comprimés dont la pression est stabilisée par des régulateurs de pression **21a** pour le gaz inerte, et **21b** pour le mélange de gaz comburant. Leur débit est réglé et contrôlé à l'aide de débitmètres massiques (type Brook) **22a** et **22b.** Des électrovannes **23a, 23b** et **23c** permettent d'inverser éventuellement les débits gazeux.

Tous les différents gaz utilisés sont purifiés et débarrassés le plus possible des traces de carbone, hydrogène, soufre et azote présentes sous forme d'impuretés organiques ou minérales.

Ils passent dans un circuit de combustion des impuretés organiques comprenant un tube de silice rempli d'oxyde de cuivre et porté à 900°C dans un four. Les composés formés sont absorbés dans des absorbeurs spécifiques **24a, 24b** :
- un tube absorbeur rempli d'amiante sodée et de perchlorate de magnésium pour piéger le dioxyde de carbone et l'eau :
- un tube absorbeur rempli de tamis moléculaire 5 Å pour parfaire le piégeage des traces d'eau résiduelles.

La zone de détection comprend, de préférence en série, un détecteur infrarouge à eau **16** relié à un détecteur infrarouge à $CO_2$ **17,** lequel est relié à un détecteur ultraviolet à $SO_2$ **18,** qui est relié à son tour à un détecteur d'oxydes d'azote 19 à chimiluminescence, ou, après piégeage de $CO_2$, $H_2O$, $SO_2$ et réduction de NO en $N_2$, à un catharomètre à détection d'azote moléculaire (variante comprise dans **19**). Les détecteurs UV et IR sont à double faisceaux non dispersifs et leur spécificité est assurée par un filtre interférentiel spécifique de la longueur d'onde d'absorption de l'élément à analyser.

Le tableau I montre l'erreur de mesure obtenue pour chaque gaz à analyser, en fonction des gaz interférants possibles. Par exemple, la détection d'anhydride carbonique subira au maximum une erreur de 0,01% en présence de 500 ppm de $SO_2$ et une erreur de 0,005% en présence de 2% d'eau. Par contre, la détection de l'azote s'effectue avec une erreur de mesure en général inférieure à 2 % que ce soit avec un catharomètre, sensible à tous les gaz interférants ou avec un détecteur à chimiluminescence.

Tableau I

| Gaz à analyser | Gaz interférant | limite de sensibilité de gaz interférant | % Erreur mesure |
|---|---|---|---|
| $CO_2$ | $SO_2$<br>$H_2O$ | 500 PPM<br>2 % | < 0.01 %<br>< 0.005 % |
| $SO_2$ | $CO_2$<br>$H_2O$ | 2 %<br>2 % | < 0.01 %<br>< 0.01 % |
| $H_2O$ | $CO_2$<br>$SO_2$ | 2 %<br>500 PPM | < 0.02 %<br>< 0.001 % |

On a vérifié que la reproductibilité s'inscrivait dans les limites d'erreurs.

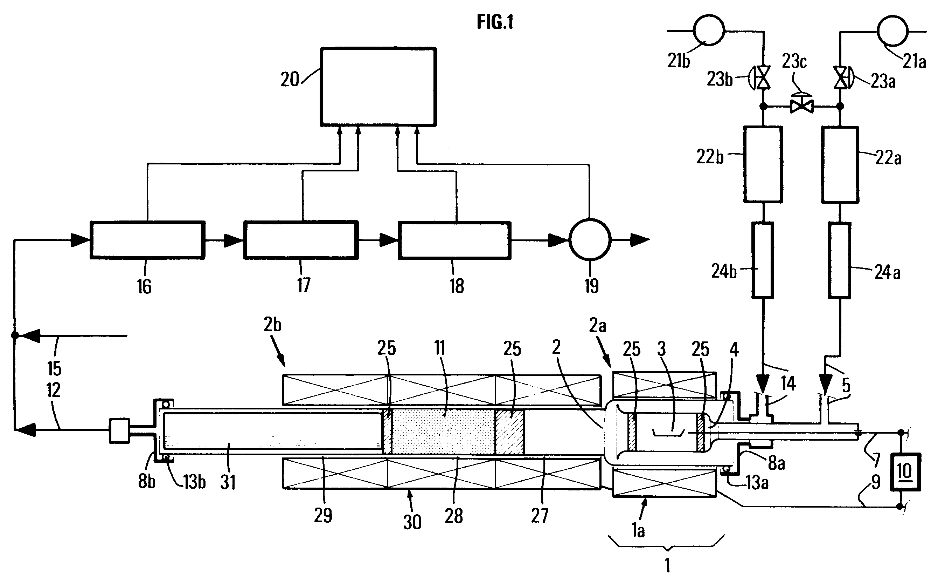

Les signaux délivrés en temps réel par chacun des détecteurs sont ensuite traités par des moyens de traitement **20** reliés à chacun des détecteurs ; ils comprennent des moyens de mémorisation des signaux, notamment ceux relatifs à la concentration instantanée d'un gaz déterminé, des moyens d'intégration des signaux en fonction du temps ou de la température, des moyens de visualisation des signaux et d'enregistrement des résultats (non représentés sur la figure **1**). Ces moyens de traitement **20** permettent également le calcul des divers paramètres tels que les rapports des valeurs $C_{/H}$, $C_{/S}$, $H_{/S}$, $C_{/N}$, $H_{/N}$ et/ou $S_{/N}$, comme on le verra ci-après dans l'exemple.

Exemple :

Un traitement d'hydroviscoréduction d'un résidu sous vide SAFANIYA 500°[+]C est réalisé en réacteur statique à 410°C sous une pression d'hydrogène de 100 bars à 410°C pendant 15 minutes.

Le résidu sous vide a les caractéristiques suivantes :

Masse volumique : 1,028 kg/l ; viscosité statique : 5900 centipoises (mPa.s) % poids d'asphaltènes ($nC_7$) : 12,4 : % poids Carbone Conradson : 22,0.

Le dispositif décrit fonctionne de la façon suivante :

L'échantillon hydrotraité (< 10 mg) est placé dans une nacelle **3** qui est introduite ensuite dans l'allonge **4**. De la laine de quartz **25** est disposée de chaque côté de la nacelle **3**. L'allonge est à son tour introduite dans la première partie **2a** de l'enceinte **2.** On isole le dispositif du milieu extérieur par les pièces **8a** et on introduit de l'hélium à un débit $d_2$ égal par exemple à 375 ml/mn dans l'allonge **4** contenue dans l'enceinte **2** par la ligne **5**, après qu'on ait purifié ce gaz. Celui-ci balaie l'allonge ainsi que le reste du dispositif.

On envoie par la ligne **14** un mélange de gaz comburant (par exemple He + $O_2$ à 3 %) à un débit $d_1$ égal par exemple à 200 ml/mn, dans la deuxième partie **2b** de l'enceinte **2** qui est chauffée par le four **30** et qui contient notamment le catalyseur d'oxyde de cuivre.

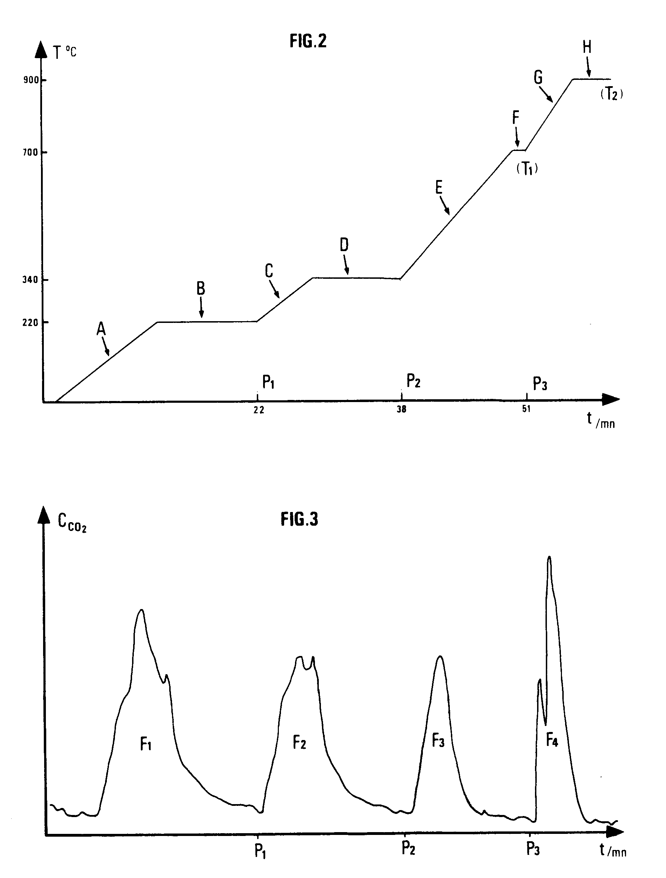

On chauffe l'enceinte **2a** par des moyens de chauffage **1a, 9**, par exemple selon des conditions opératoires définies par la figure 2, jusqu'à la température $T_1$ choisie par exemple à 700°C, conditions qui sont les suivantes :

$V_A = V_C = 20$°C/mn
$V_E = 30$°C/mn
$V_G = 100$°C/mn
$t_A = 11$ mn , $t_B = 10$ mn , $t_C = 6$ mn , $t_D = 10$ mn
$t_E = 12$ mn , $t_F = 1$ mn , $t_G = 2$ mn , $t_H = 2$ mn

L'effluent gazeux de la fraction vaporisée traverse la zone **27** portée à une température par exemple de 750°C, puis la zone catalytique **28** à 850°C, enfin la dernière zone **29** d'oxydation à 950°C.

L'effluent gazeux de la fraction volatile à la température $T_1$ est ainsi oxydé et transformé en $CO_2$, $SO_2$, $H_2O$. Un débit supplémentaire d'hélium égal par exemple à 100 ml/mn peut être apporté par la ligne **15** en sortie **12** du tube **2b** et favorise le transfert des premiers composés d'oxydation (ceux de la fraction volatile) dans les différents détecteurs, et notamment les détecteurs **16**, **17** et **18** de $H_2O$, $CO_2$ et $SO_2$ qui les détectent ainsi de manière sensiblement simultanée en temps réel, en fonction du paramètre désiré (température de chauffage par exemple).

On procède ensuite à la deuxième étape du procédé.

On arrête l'introduction d'hélium dans l'allonge **4** et on envoie à la place de l'hélium le mélange de gaz comburant à l'intérieur de l'allonge **4** après avoir commuté le circuit de gaz comburant par l'intermédiaire des électrovannes **23** et **23c** dans la ligne **5**. On continue d'alimenter le tube **2b** en mélange de gaz comburant par la ligne **14** de façon que le débit total équivalent à $d_1$ + $d_2$ préconisé dans la première étape du procédé reste sensiblement le même.

On porte l'enceinte **2a** à une température $T_2$ égale par exemple à 900°C selon les conditions de la figure 2, et on oxyde totalement le résidu de l'échantillon. Le produit d'oxydation du résidu passe ensuite comme il a été dit précédemment pour l'effluent gazeux dans la zone d'oxydation **28** contenant l'oxyde de Cu pour convertir notamment le monoxyde de carbone en anhydride carbonique ; cette zone d'oxydation est à une température sensiblement identique à celle obtenue lors de la combustion de la fraction volatile ; les concentrations instantanées des composés d'oxydation (les seconds) du résidu sont détectées de manière sensiblement simultanée en temps réel et les signaux analysés comme précédemment.

Les concentrations instantanées en $CO_2$, $H_2O$ et $SO_2$, en fonction du temps sont indiquées sur les figures **3**, **4** et **5**.

Sur ces figures, ont été aussi indiqués les points $P_1$ et $P_2$ correspondant aux n-alcanes chauffés dans les mêmes conditions et dont les points d'ébullition réels sont respectivement égaux à 500°C et 620°C. On peut considérer qu'à la fin du palier isotherme D, va s'achever sensiblement la zone de distillation et vaporisation proprement dite et qu'au-delà, va se superposer une zone de distillation et craquage jusqu'au point $P_3$, à partir duquel on oxyde le résidu.

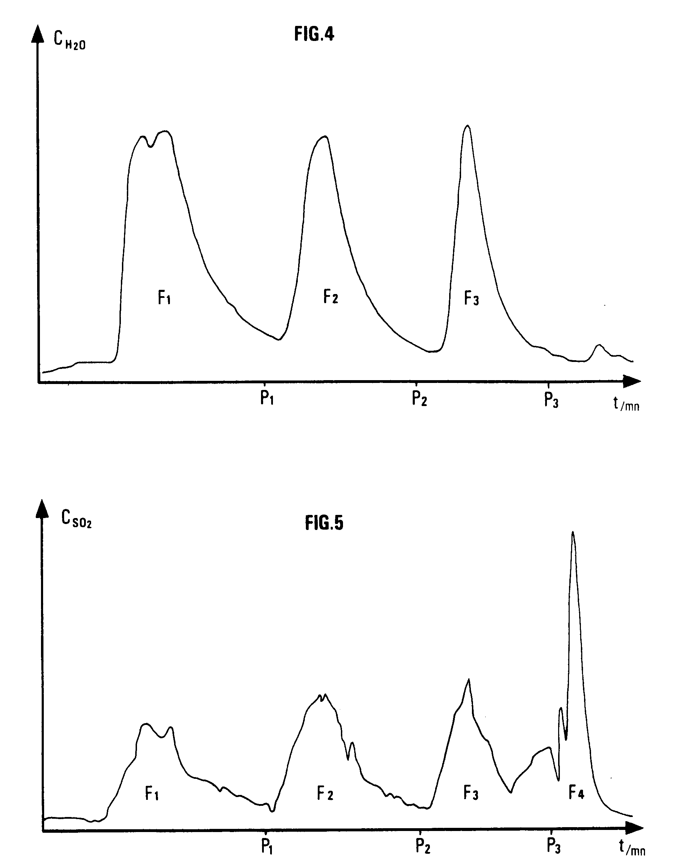

Le tableau II présente des résultats analytiques relatifs à 4 fractions $F_1$, $F_2$, $F_3$, $F_4$. A partir des courbes de distribution ($CO_2$, Fig. 3 : $H_2O$, Fig. 4 : $SO_2$, Fig 5), on peut calculer par exemple :

- le pourcentage des diverses fractions, obtenu par intégration des surfaces de chaque courbe, la surface totale obtenue représentant 100 %,
- la teneur respective en C, H, S, calculée de la façon suivante, par exemple pour le carbone :
  . Poids en mg de l'échantillon : p
  . Surface totale de la courbe ($CO_2$, Fig. 3) : $S_c$ unités de surface,
  . Etalonnage ou coefficient de réponse : 1 mg carbone correspond à $s_c$ unités de surface,
  d'où :
  . Poids de carbone dans l'échantillon :

$$P_C = \frac{S_c}{s_c}$$

soit une teneur totale en carbone :

$$C \% = \frac{P_c}{p} \times 100$$

Connaissant la teneur totale en carbone C %, on peut calculer la teneur en carbone dans chaque fraction $C_F$.

On peut de la même façon calculer S % et $S_F$ ainsi que H % et $H_F$, et à partir de ces résultats calculer également par exemple le rapport atomique $H_{/C}$, $S_{/C}$, ce qui donnera pour la fraction $F_1$ :

$$\frac{H}{C} (F_1) = \frac{H_{F_1}}{\dfrac{C_{F_1}}{12}} = \frac{0,44 \times 10,2}{\dfrac{0,394 \times 84}{12}} = 1,627$$

$$\frac{S}{C} (F_1) = \frac{\dfrac{S_{F_1}}{32}}{\dfrac{C_{F_1}}{12}} = 0,011$$

Tableau II

| Hydroviscoréduction du résidu sous vide SAFANIYA | | | | | |
|---|---|---|---|---|---|
| | % C | % H | % S | $H_{/C}$ atomique | $S_{/C}$ atomique |
| $F_1$ 500$^-$ | 39,4 | 44,0 | 24,5 | 1,627 | 0,011 |
| $F_2$ 500 - 620 | 28,6 | 32,3 | 28,65 | 1,646 | 0,018 |
| $F_3$ Fin de distillation | 15,0 | 21,7 | 27,85 | 2,108 | 0,033 |
| $F_4$ Résidu | 17,0 | 2,0 | 19,0 | 0,171 | 0,020 |
| TOTAL % | 100 | 100 | 100 | | |
| Teneur totale % poids | 84,00 | 10,2 | 3,97 | 1,46 | 0,018 |

Pour comparaison, on a présenté dans le tableau III les caractéristiques du résidu sous vide non traité obtenues selon l'invention, dans les mêmes conditions opératoires.

Tableau III

| Résidu sous vide SAFANIYA (Arabe Lourd) | | | | | |
|---|---|---|---|---|---|
| | % C | % H | % S | $H_{/C}$ atomique | $S_{/C}$ atomique |
| $F_1$ % 500⁻ | 6,5 | 3,0 | 3,2 | 0,69 | 0,011 |
| $F_2$ % 500 - 620 | 36,3 | 42,2 | 26,3 | 1,75 | 0,015 |
| $F_3$ fin de distillation | 42,8 | 52,3 | 48,7 | 1,84 | 0,025 |
| $F_4$ Résidu | 14,4 | 2,5 | 21,8 | 0,25 | 0,032 |
| TOTAL | 100 | 100 | 100 | | |
| Teneur totale en % poids | 83,25 | 10,43 | 4,73 | 1,50 | 0,021 |

**Revendications**

1. Procédé permettant de déterminer les teneurs d'au moins deux éléments choisis parmi l'hydrogène, le carbone, le soufre et l'azote contenus dans au moins deux fractions d'un échantillon de matière organique, comprenant une étape de chauffage sous flux d'atmosphère inerte à une température $T_1$ au plus égale à 700° C dans une première zone de chauffage, de façon à réaliser une étape de vaporisation-distillation délivrant un effluent gazeux de la fraction vaporisée et un résidu, une étape de combustion dudit effluent gazeux en présence d'un catalyseur d'oxydation dans une zone de combustion et une étape de combustion du résidu par un gaz contenant de l'oxygène moléculaire délivrant en présence d'un catalyseur d'oxydation un produit d'oxydation dudit résidu, ledit procédé est caractérisé en ce que :

   a) on effectue ladite étape de combustion dudit effluent gazeux en présence d'un gaz pauvre en oxygène dans des conditions telles que l'on forme des premiers composés d'oxydation desdits éléments contenus dans ledit effluent et comprenant essentiellement de l'anhydride carbonique, de la vapeur d'eau, de l'anhydride sulfureux et/ou de l'oxyde d'azote, on détecte de manière sensiblement simultanée au moyen de détecteurs spécifiques deux au moins desdits premiers signaux représentatifs desdits premiers composés d'oxydation.

   b) on effectue ladite étape de combustion dudit résidu par un gaz pauvre en oxygène moléculaire dans des conditions telles que l'on forme des secondes composés d'oxydation desdits éléments contenus dans ledit produit d'oxydation et comprenant essentiellement de l'anhydride carbonique, de la vapeur d'eau, de l'anhydride sulfureux et de l'oxyde d'azote, et on détecte de manière sensiblement simultanée au moyen desdits détecteurs deux au moins des seconds composés d'oxydation contenus dans ledit produit d'oxydation du résidu de façon à obtenir des seconds signaux représentatifs desdits seconds composés, et

   c) on déduit desdits premiers et seconds signaux lesdites teneurs d'au moins deux éléments respectivement de la fraction vaporisée et du résidu.

2. Procédé selon la revendication 1 dans lequel ledit gaz contient une quantité d'oxygène comprise entre environ 1 à 15 % en volume.

3. Procédé selon la revendication 2 dans lequel ledit gaz est un mélange de gaz inerte sec et d'oxygène sensiblement pur.

4. Procédé selon l'une des revendications 1 à 3 dans lequel on fait s'écouler ladite atmosphère inerte dans la première zone de chauffage à un débit $d_2$ compris entre 100 et 1000 ml/mn et ledit gaz pauvre en oxygène dans ladite zone de combustion à un débit $d_1$ compris entre 50 et 400 ml/mn de sorte qu'il s'écoule un débit de gaz ($d_2$ + $d_1$) dans ladite zone de combustion durant l'étape de combustion dudit effluent gazeux et dans lequel on fait s'écouler sensiblement le même débit ($d_2$ + $d_1$) de gaz consistant essentiellement en gaz pauvre en oxygène dans la zone de combustion durant l'étape de combustion dudit résidu.

5. Procédé selon l'une des revendications 1 à 4 dans lequel ladite zone de combustion est soumise à un gradient de température positif.

6. Procédé selon l'une quelconque des revendications 1 à 5 dans lequel la valeur de ladite température $T_1$ est maintenue constante durant l'étape de vaporisation-distillation.

7. Procédé selon l'une quelconque des revendications 1 à 5 dans lequel on chauffe ledit échantillon sous atmosphère inerte à une vitesse d'augmentation de la température comprise entre 1 et 100°C/mn et de préférence entre 10 et 50°C/mn.

8. Procédé selon l'une quelconque des revendications 1 à 7 dans lequel on effectue la combustion dudit résidu à une température $T_2$ au plus égale à 1100°C.

9. Procédé selon l'une quelconque des revendications 1 à 8 dans lequel on détecte au moins deux signaux représentatifs desdits premiers composés d'oxydation d'au moins un élément choisi parmi le carbone, l'hydrogène, le soufre et l'azote, en fonction d'au moins deux intervalles de temps successifs ou de température d'ébullition de composés de référence chauffés selon l'étape a) .

10. Procédé selon l'une quelconque des revendications 1 à 9 dans lequel on détermine les valeurs des rapports desdits éléments $C_{/H}$, $C_{/S}$, $H_{/S}$, $C_{/N}$, $H_{/N}$ et/ou $S_{/N}$ ou leurs inverses en fonction de la température de chauffage ou d'ébullition de composés de référence.

11. Dispositif pour la mise en oeuvre du procédé selon l'une quelconque des revendications 1 à 10, caractérisé en ce qu'il comprend en combinaison :
   - une enceinte **(2)**,
   - des moyens de chauffage **(1)** de ladite enceinte,
   - des moyens de support **(3)** dudit échantillon dans ladite enceinte,
   - des moyens d'alimentation **(21a, 22a, 24, 5)** en gaz inerte reliés à ladite enceinte qui sont adaptés à produire en combinaison avec les moyens de chauffage ledit effluent gazeux de la fraction vaporisée,
   - des moyens d'alimentation **(21b, 22b, 24b, 14)** en mélange de gaz comburant reliés à des moyens de combustion définis ci-dessous,
   - des moyens de combustion **(30, 2b)** reliés à ladite enceinte **(2)** et adaptés d'une part à délivrer lesdits premiers composés d'oxydation desdits éléments à partir de l'effluent gazeux et, d'autre part, à délivrer lesdits seconds composés d'oxydation à partir dudit produit d'oxydation du résidu,
   - des moyens d'alimentation **(21b, 22a, 24a, 5)** en mélange de gaz comburant reliés à ladite enceinte qui sont adaptés à produire ledit produit d'oxydation du résidu,
   - des moyens de détection spécifique **(16, 17, 18, 19)** adaptés à détecter simultanément deux au moins desdits premiers composés d'oxydation, puis deux au moins desdits seconds composés d'oxydation, et reliés auxdits moyens de combustion **(2b, 30)**, et
   - des moyens de traitement **(20)** reliés auxdits moyens de détection et adaptés à traiter lesdits premiers et seconds signaux.

12. Dispositif selon la revendication 11 dans lequel lesdits moyens de chauffage **(1, 10)**, comprennent un premier four **(1a)** et des moyens de contrôle **(7)**, de régulation et de programmation **(9)** de température, connectés audit four.

13. Dispositif selon l'une quelconque des revendications 11 et 12 dans lequel lesdits moyens d'alimentation en gaz inerte **(22a, 24a)** et lesdits moyens d'alimentation en mélange de gaz oxydant **(22b, 24b)** comprennent des moyens de régulation **(22a, 22b)** massiques de débit de gaz reliés à une source de gaz et des moyens de purification **(24a, 24b)** desdits gaz reliés d'une part auxdits moyens de régulation massique, et d'autre part auxdits moyens de chauffage.

14. Dispositif selon l'une quelconque des revendications 11 à 13, dans lequel lesdits moyens de combustion **(30, 2b)** comprennent une deuxième enceinte **(2b)** renfermant un catalyseur d'oxydation **(11)** et des seconds moyens de chauffage **(30, 10)** de la deuxième enceinte **(2b).**

15. Dispositif selon l'une quelconque des revendications 11 à 14 dans lequel lesdits moyens de détection **(16, 17, 18, 19)** comprennent au moins un détecteur spécifique choisi dans le groupe formé par le

EP 0 269 511 B1

détecteur à vapeur d'eau **(16)**, le détecteur à anhydride carbonique **(17),** le détecteur à anhydride sulfureux **(18)** et le détecteur à oxyde d'azote **(19)** ou à azote.

16. Dispositif selon l'une des revendications 11 à 15, dans lequel les moyens de détection **(16, 17, 18, 19)** sont montés en série.

17. Dispositif selon l'une quelconque des revendications 15 et 16 dans lequel le détecteur à vapeur d'eau **(16)** est connecté à la sortie de moyens de combustion **(2b, 30).**

18. Dispositif selon l'une quelconque des revendications 11 à 17 dans lequel les moyens de détection comprennent un détecteur IR à eau, un détecteur IR à $CO_2$, un détecteur UV à anhydride sulfureux, et un détecteur à chimiluminescence ou un catharomètre.

**Claims**

1. A process for determining the amounts of at least two elements selected from hydrogen, carbon, sulfur and nitrogen contained in at least two fractions of a sample of organic matter, comprising a heating step under inert atmosphere stream at a temperature $T_1$ of at most $700°C$ in a first heating zone, so as to perform a vaporization-distillation step providing a gas effluent of the vaporized fraction and a residue, a step of combustion of said gas effluent in the presence of an oxidation catalyst in a combustion zone and a step of combustion of the residue by means of a molecular oxygen-containing gas providing, in the presence of an oxidation catalyst, an oxidation product of said residue, said process being characterized in that :

   a) said combustion step of the gas effluent is performed in the presence of a gas of low oxygen content in such conditions as to form first oxidation compounds of said elements contained in said effluent and comprising essentially carbon dioxide, steam, sulfur dioxide and/or nitrogen oxide, and at least two of said first signals representative of said first oxidation compounds are substantially simultaneously detected by means of specific detectors.

   b) said combustion step of said residue, by means of a gas of low molecular oxygen content, is performed in such conditions as to form second oxidation compounds of said elements contained in said oxidation product and comprising essentially carbon dioxide, steam, sulfur dioxide and nitrogen oxide, and at least two of said second oxidation compounds contained in said residue oxidation product are substantially simultaneously detected by means of said detectors so as to obtain second signals representative of said second compounds, and

   c) said first and second signals are used to deduce said contents of at least two elements, respectively of the vaporized fraction and of the residue.

2. A process according to claim 1, wherein said gas contains an oxygen amount from about 1 to 15% by volume.

3. A process according to claim 2, wherein said gas is a mixture of dry inert gas with substantially pure oxygen.

4. A process according to one of claims 1 to 3, wherein said inert atmosphere is fed to said first heating zone at a rate $d_2$ from 100 to 100ml/mn and said gas of low oxygen content is fed to said combustion zone at a rate $d_1$ from 50 to 400 ml/mn, so that the total gas flow rate is $d_1 + d_2$ in said combustion zone during the combustion step of said gas effluent, substantially the same total flow rate ($d_1 + d_2$) essentially of gas of low oxygen content being fed to the combustion zone during said residue combustion step.

5. A process according to one of claims 1 to 4 , wherein the combustion zone is subjected to a positive temperature gradient.

6. A process according to any one of claims 1 to 5, wherein said temperature $T_1$ is maintained constant during the vaporization-distillation step.

7. A process according to any one of claims 1 to 5, wherein said sample is heated under inert atmosphere with a temperature rate of increase from 1 to $100°C/mn$, preferably from 10 to $50°C/mn$.

12

**8.** A process according to any one of claims 1 to 7, wherein the combustion of said residue is performed at a temperature $T_2$ of at most 1100°C.

**9.** A process according to any one of claims 1 to 8, wherein at least two signals representative of said first oxidation compounds of at least one element selected from carbon, hydrogen, sulfur and nitrogen, are detected versus at least two successive time or boiling temperature intervals of reference compounds heated according to step a).

**10.** A process according to any one of claims 1 to 9, wherein the values of $C_{/H}$, $C_{/S}$, $H_{/S}$, $C_{/N}$, $H_{/N}$ and/or $S_{/N}$ ratios of said elements, or the inverse values, are determined versus the heating or boiling temperature of reference compounds.

**11.** An apparatus for carrying out the process according to any one of claims 1 to 10, characterized by the combination of :
- an enclosure (2),
- means (1) for heating said enclosure,
- support means (3) for said sample, in said enclosure,
- inert gas feeding means (21a, 22a, 24a, 5) connected to said enclosure, adapted to produce, in combination with the heating means, said vaporized fraction gas effluent,
- combustion-sustaining gas mixture feeding means (21b, 22b, 24b, 14) connected to hereinafter-defined combustion means,
- combustion means (30, 2b) connected to said enclosure (2) and adapted to deliver said first oxidation compounds of said elements from the gas effluent and also to deliver said second oxidation compounds from said residue oxidation product,
- combustion-sustaining gas mixture feeding means (21b, 22b, 24a 5) connected to said enclosure, adapted to produce said residue oxidation product,
- specific detection means (16, 17, 18, 19) adapted to simultaneously detect at least two of said first oxidation compounds, then at least two of said second oxidation compounds, and connected to said combustion means (2b, 30), and
- processing means (20) connected to said detection means and adapted to process said first and second signals.

**12.** An apparatus according to claim 11, wherein said heating means (1, 10), comprises a first furnace (1a), temperature control means (7) and regulation and programmation means (9) connected to said furnace.

**13.** An apparatus according to any one of claims 11 and 12, wherein said inert gas feeding means (22a, 24a) arid said oxidizing gas mixture feeding means (22b, 24b) comprise gas flow rate mass regulation means (22a, 22b) connected to a gas source and purification means (24a, 24b) for said gases connected both to said mass regulation means and to said heating means.

**14.** An apparatus according to any one of claims 11 to 13, wherein said combustion means (30, 2b) comprises a second enclosure (2b) containing an oxidation catalyst (11) and second means (30, 10) for heating said second enclosure (2b).

**15.** An apparatus according to any one of claims 11 to 14, wherein said detection means (16, 17, 18, 19) comprise at least one specific detector selected from the group consisting of steam detector (16), carbon dioxide detector (17), sulfur dioxide detector (18) and nitrogen oxide or nitrogen detector (19).

**16.** An apparatus according to any one of claims 11 to 15, wherein the detection means (16, 17, 18, 19) are serially mounted.

**17.** An apparatus according to any one of claims 15 and 16, wherein the steam detector (16) is connected to the output of combustion means (2b, 30).

**18.** An apparatus according to any one of claims 11 to 17, wherein the detection means comprise an IR water detector, an IR $CO_2$ detector, a sulfur dioxide UV detector and a chemiluminescence detector or a katharometer.

## EP 0 269 511 B1

**Patentansprüche**

1. Verfahren zum Bestimmen des Gehaltes von mindestens zwei Elementen, ausgewählt aus Wasserstoff, Kohlenstoff, Schwefel und Stickstoff, die in wenigstens zwei Fraktionen einer Probe aus organischem Stoff enthalten sind, umfassend einen Heizschritt unter Inertatmosphäre bei einer Temperatur $T_1$ von höchstens gleich 700 °C in einer ersten Heizzone, derart, um einen verdampfungs-Destillationsschritt durchzuführen, welcher einen gasförmigen Abgang der verdampften Fraktion und einen Rückstand liefert, einen Verbrennungsschritt dieses gasförmigen Abganges in Anwesenheit eines Oxidationskatalysators in einer Verbrennungszone und einen Verbrennungsschritt des Rückstandes durch ein Gas, das molekularen Sauerstoff umfaßt, welcher in Anwesenheit eines Oxidationskatalysators ein Oxidationsprodukt dieses Rückstandes liefert, dadurch gekennzeichnet, daß:
   a) man diesen Verbrennungsschritt dieses gasförmigen Abganges in Anwesenheit eines sauerstoffarmen Gases unter solchen Bedingungen durchführt, daß man erste Oxidationszusammensetzungen dieser Elemente, welche in diesem Abgang umfaßt sind und im wesentlichen Kohlendioxid, Wasserdampf, Schwefeldioxid und/oder Stickoxid umfassen, bildet, man mittels spezifischer Detektoren zwei wenigstens dieser ersten typischen Signale dieser ersten Oxidationszusammensetzungen auf merklich gleichzeitiger Weise ermittelt,
   b) man diesen Verbrennungsschritt dieses Rückstandes durch ein an molekularem Sauerstoff armes Gas unter solchen Bedingungen durchführt, daß man zweite Oxidationszusammensetzungen dieser Elemente bildet, welche in diesem Oxidationsprodukt umfaßt sind und im wesentlichen Kohlendioxid, Wasserdampf, Schwefeldioxid und Stickoxid umfassen, und man mittels dieser Detektoren zwei wenigstens der zweiten Oxidationszusammensetzungen, die in diesem Oxidationsprodukt des Rückstandes enthalten sind, auf merklich gleichzeitige Weise ermittelt, um zweite typische Signale dieser zweiten Zusammensetzungen zu erhalten, und
   c) man von diesen ersten und zweiten Signalen diese Gehalte von wenigstens zwei Elementen jeweils der verdampften Fraktion und des Rückstandes ableitet.

2. Verfahren nach Anspruch 1, dadurch gekennzeichnet, daß dieses Gas eine Menge Sauerstoff zwischen 1 bis 15 Vol.-% einschließlich enthält.

3. Verfahren nach Anspruch 2, dadurch gekennzeichnet, dieses gas ein gemisch austrockenem Inertgas und merklich reinem Sauerstoff ist.

4. Verfahren nach einem der Ansprüche 1 bis 3, dadurch gekennzeichnet, daß man diese Inertatmosphäre in der ersten Verbrennungszone mit einem Durchsatz $d_1$ zwischen 100 und 1000 ml/min und dieses sauerstoffarme Gas in dieser Verbrennungszone mit einem Durchsatz $d_2$ zwischen 50 und 400 ml/min einschließlich strömen läßt, derart, daß ein Gasdurchsatz ($d_2$ + $d_1$) in dieser Verbrennungszone während des Verbrennungsschrittes dieses gasförmigen Abganges fließt und man in diesem merklich den gleichen Durchsatz ($d_2$ + $d_1$) an Gas, das im wesentlichen sauerstoffarmes Gas umfaßt, in der Verbrennungszone während des Verbrennungsschrittes dieses Rückstandes strömen läßt.

5. Verfahren nach einem der Ansprüche 1 bis 4, dadurch gekennzeichnet, daß diese Verbrennungszone einem positiven Temperaturgradienten unterworfen wird.

6. Verfahren nach einem der Ansprüche 1 bis 5, dadurch gekennzeichnet, daß der Wert dieser Temperatur $T_1$ während des Verdampfungs-Destillationsschrittes konstant gehalten wird.

7. Verfahren nach einem der Ansprüche 1 bis 5, dadurch gekennzeichnet, daß man diese Probe unter Inertatmosphäre mit einer Temperaturerhöhungsgeschwindigkeit zwischen 1 und 100 °C/min einschließlich und vorzugsweise zwischen 10 und 50 °C aufheizt.

8. Verfahren nach einem der Ansprüche 1 bis 7, dadurch gekennzeichnet, daß man die Verbrennung dieses Rückstandes bei einer Temperatur $T_2$ von höchstens gleich 1100 °C durchführt.

9. Verfahren nach einem der Ansprüche 1 bis 8, dadurch gekennzeichnet, daß man wenigstens zwei typische Signale dieser ersten Oxidationszusammensetzungen von mindestens einem Element, ausgewählt aus Kohlenstoff, Wasserstoff, Schwefel und Stickstoff, in Funktion von wenigstens zwei aufeinanderfolgenden Zeitintervallen oder der Siedetemperatur von Vergleichszusammensetzungen, die gemäß

14

EP 0 269 511 B1

Schritt a) aufgeheizt sind, ermittelt.

**10.** Verfahren nach einem der Ansprüche 1 bis 9, dadurch gekennzeichnet, daß man die Bezugswerte dieser Elemente $C_{/H}$, $C_{/S}$, $H_{/S}$, $C_{/N}$, $H_{/N}$ und/oder $S_{/N}$ oder ihre Kehrwerte in Funktion der Heiztemperaturen oder Siedetemperatur der Vergleichszusammensetzungen bestimmt.

**11.** Vorrichtung zur Durchführung des Verfahrens nach einem der Ansprüche 1 bis 10, dadurch gekennzeichnet, daß sie in Kombination umfaßt:
- eine Einfassung (2),
- Heizmittel (1) dieser Einfassung,
- Trägermittel (3) dieser Probe in dieser Einfassung,
- mit dieser Einfassung verbundene Versorgungsmittel (21a, 22a, 24, 5) für Inertgas, die geeignet sind, diesen gasförmigen Abgang der verdampften Fraktion in Kombination mit diesen Heizmitteln zu erzeugen,
- Versorgungsmittel (21b, 22b, 24b, 14) für die Verbrennung bewirkendes Gasgemisch, das mit den unten definierten Verbrennungsmitteln verbunden ist,
- Verbrennungsmittel (30, 2b), die mit dieser Einfassung (2) verbunden und geeignet sind, einerseits diese ersten Oxidationszusammensetzungen dieser Elemente auf der Grundlage von dem gasförmigen Abgang zu liefern und andererseits diese zweiten Oxidationszusammensetzungen auf der Grundlage von diesem Oxidationsprodukt des Rückstandes zu liefern,
- mit dieser Einfassung verbundene Versorgungsmittel (21b, 22a, 24a, 5) für die Verbrennung bewirkendes Gasgemisch, die geeignet sind, dieses Oxidationsprodukt des Rückstandes zu erzeugen,
- spezifische Detektionsmittel (16, 17, 18, 19), die geeignet sind, zwei wenigstens dieser ersten Oxidationszusammensetzungen, dann zwei wenigstens der zweiten Oxidationszusammensetzungen zu ermitteln, und mit diesen Verbrennungsmitteln (2b, 30) verbunden sind, und
- Verarbeitungsmittel (20), die mit diesen Detektionsmitteln verbunden und geeignet sind, diese ersten und zweiten Signale zu verarbeiten.

**12.** Vorrichtung nach Anspruch 11, dadurch gekennzeichnet, daß diese Heizmittel (1, 10) einen ersten Ofen und mit diesem Ofen verbundene Steuerungs- bzw. Regelungsmittel (7) zur Regelung und Programmierung (9) der Temperatur umfassen.

**13.** Vorrichtung nach einem der Ansprüche 11 und 12, dadurch gekennzeichnet, daß diese Versorgungsmittel (22a, 24a) für Inertgas und diese Versorgungsmittel (22b, 24b) für oxidierendes Gasgemisch Massenregelungsmittel (22a, 22b) für den Gasdurchsatz, welche mit einer Gasquelle verbunden sind, und Reinigungsmittel (24a, 24b) dieser Gase, welche mit einerseits diesen Massenregelungsmitteln und andererseits diesen Heizmitteln verbunden sind, umfassen.

**14.** Vorrichtung nach einem der Ansprüche 11 bis 13, dadurch gekennzeichnet, daß diese Verbrennungsmittel (30,2b) eine zweite Einfassung (2b) umfassen, die einen Oxidationskatalysator und zweite Heizmittel (30, 10) der zweiten Einfassung (2b) einschließt.

**15.** Vorrichtung nach einem der Ansprüche 11 bis 14, dadurch gekennzeichnet, daß diese Detektionsmittel (16, 17, 18, 19) wenigstens einen spezifischen Detektor, ausgewählt aus der durch den Wasserdampfdetektor (16), den Kohlendioxiddetektor (17), den Schwefeldioxiddetektor (18) und den Stickoxid- oder Stickstoffdetektor (19) gebildeten Gruppe umfassen.

**16.** Vorrichtung nach einem der Ansprüche 11 bis 15, dadurch gekennzeichnet, daß die Detektionsmittel (16, 17, 18, 19) in Reihe angeordnet sind.

**17.** Vorrichtung nach einem der Ansprüche 11 bis 16, dadurch gekennzeichnet, daß der Wasserdampfdetektor (16) mit dem Ausgang der Heizmittel (2b, 30) verbunden ist.

**18.** Vorrichtung nach einem der Ansprüche 11 bis 17, dadurch gekennzeichnet, daß die Detektionsmittel einen IR-Detektor für Wasserdampf, einen IR-Detektor für Kohlendioxid, einen UV-Detektor für Schwefeldioxid und einen Detektor für Chemolumineszenz oder ein Katharometer (catharomètre) umfassen.

15

**FIG.1**

EP 0 269 511 B1

FIG.2

FIG.3

FIG.4

FIG.5